# EUROPÄISCHE PATENTANMELDUNG

(11) **EP 0 838 171 A2**
(43) Veröffentlichungstag der Anmeldung: **29.04.1998**
(21) Anmeldenummer: 97250287.6
(22) Anmeldetag: 24.09.1997
(51) Int. Cl.: A43B 21/32

(54) **Fersensporn-Bettung**

(30) Priorität: 25.09.1996 DE 29617374 U
(71) Anmelder: Schelchen GmbH, 15711 Zeesen (DE)
(72) Erfinder: Schelchen, Hartmut, 13465 Berlin (DE); Timm, Thomas, 12309 Berlin (DE)
(74) Vertreter: Christiansen, Henning, Dipl.-Ing.

(57) **Zusammenfassung**

Die Erfindung betrifft eine Fersensporn-Bettung (1, 1') als Teil eines Fersenkissens (2, 2a, 2b, 2c, 2d, 22.1), welches seinerseits gegebenenfalls Teil des Fußbetts einer orthopädischen Einlage oder eines Schuhs ist, bestehend aus einem elastischen Werkstoff, welches zur mechanischen Entlastung eines Teilbereichs der Ferse eine Ausnehmung (3, 3a, 3b, 3c, 3d) aufweist, in welche ein Polster (5, 5', 5'') zur Aufnahme des Fersensporns eingefügt ist, wobei der Werkstoff des Fersenkissens bei Druckbelastung nachgiebiger ist, als der an das Polster angrenzende Werkstoff. Die Außenabmessungen des Polsters (5, 5', 5'') bzw. der Ausnehmung (3, 3a, 3b, 3c, 3d) sind in Längsrichtung des Fußes größer als in Querrichtung, wobei der vordere Teil der Ausnehmung zur Fußinnenseite hin weist, in der Weise, daß mindestens der überwiegende Teil der Fersenspornausbildungen mit einer einzigen und geschlossenen geometrischen Form der Ausnehmung druckentlastend erfaßbar ist.

## Beschreibung

Die Erfindung betrifft eine Fersensporn-Bettung der im Oberbegriff des Anspruchs 1 angegebenen Art.

Ein Fersensporn ist eine dornartige, knöcherne Ausziehung an der Unterseite des Fersenbeinhöckers, welche hauptsächlich am Ansatzpunkt von Sehnen und/oder Aponeurosenfasern durch lokale mechanische Überlastung entsteht. Für eine Therapie der Entzündung wird eine mechanische Entlastung des Fersensporns angestrebt. Dazu ist eine Fersensporn-Bettung mit einem Fersenkissen bekannt.

Die bekannte Fersenspornbettung besteht aus einem nachgiebigen Material, welches auf seiner der Ferse zugewandten Seite mit einem vorzugsweise aus Leder bestehenden Bezug versehen ist. Das Fersenkissen ist in Richtung des Randes abgeflacht und weist in Mittenposition eine im wesentlichen zylindrisch ausgebildete Ausnehmung auf, in welche eine Fersenpolster aus weicherem Material eingesetzt ist und welche von dem Bezug des Fersenkissens überspannt wird. Die bekannte Fersenspornbettung wird von einem Orthopädiemechaniker jeweils individuell an die vorgefundenen Verhältnisse geometrisch angepaßt.

Diese Einzelanfertigung ist kosten- und zeitaufwendig.

Ausgehend von den Mängeln des Standes der Technik liegt der Erfindung die Aufgabe zugrunde, eine Fersensporn-Bettung der eingangs genannten Gattung anzugeben, welche einheitlich ohne zusätzliche Anpaßarbeiten zur Behandlung einer größeren Zahl von Patienten einsetzbar ist und damit kostengünstig hergestellt werden kann.

Diese Aufgabe wird mit den kennzeichnenden Merkmalen des Anspruchs 1 gelöst.

Die Erfindung schließt die Erkenntnis ein, daß der weicher abzufedernde Druckbereich der meisten Fälle der Fersensporn-Therapie in einen abgegrenzten Bereich fällt, der gegenüber dem bekannten kreisförmig umrandeten Bereich länglich (elliptisch oder oval) erweitert ist. Wenn man nun von vorn herein bei einer einheitlich gestalteten Auflagefläche einen einheitliche weicher elastisch gepolsterte Druckentlastungsbereich vorsieht, der alle diese Fälle einschließt, ist eine individuelle Nachbearbeitung in der weitaus überwiegenden Zahl der Fälle entbehrlich. Industriell vorgefertigte Fersenbettungen können ohne Nacharbeit verwendet werden, um den individuellen Fällen gerecht zu werden, da eine länglich erweiterte, weicher elastisch gestaltete Druckauflagefläche therapeutisch keinen Nachteil gegenüber einer genau angepaßten kreisflächenförmigen Auflagefläche beinhaltet.

Entsprechend der bevorzugten Ausführungsform der Erfindung weist die Fersensporn-Bettung ein an die Anatomie der menschlichen Ferse angepaßtes Fersenkissen aus einem elastischen Werkstoff auf, welches zur mechanischen Entlastung von Teilbereichen der Ferse mit einer das Fersenkissen durchdringende Ausnehmung versehen ist. In diese Ausnehmung ist ein Fersenpolster formschlüssig eingesetzt, welches weicher ausgebildet ist als das Fersenkissen.

Bevorzugt weist das Fersenpolster bzw. die Ausnehmung eine symmetrisch verrundete, insbesondere elliptische, Grundfläche auf, wobei das Verhältnis der Länge der großen Achse zur kleinen Achse der Ellipse einen Wert zwischen 1,4 bis 2,0, vorzugsweise 1,7, aufweist.

Gleichzeitig ist bei einer ellipsenartigen oder ovalen Ausgestaltung der Ausnehmung bzw. des Polsters der vorn gelegene Brennpunkt der Ellipse bzw. Mittelpunkt des Halbkreises des Ovals zur Fußinnenseite hin versetzt angeordnet.

Dabei schneidet die große Achse der Ellipse bzw. Längsachse des Ovals die Fußmittelachse im Bereich ihres hinteren Viertels, vorzugsweise bei im wesentlichen 20% ihrer Länge, der Schnittpunkt zwischen Fußmittelachse und großer Achse der Ellipse bzw. Längsachse des Ovals ist vom hinteren Ende des Fußes im wesentlichen um die Länge der großen Achse der Ellipse bzw. das Maß der Längserstreckung des Ovals nach vorn versetzt angeordnet und die große Achse der Ellipse bzw. die Längsachse des Ovals und die Mittelachse des Fußes schließen einen Winkel von 35° bis 55°, vorzugsweise von 45°, ein.

Eine derartige Anordnung und Ausbildung der Ausnehmung bzw. des Fersenpolsters der erfindungsgemäßen Fersensporn-Bettung ermöglicht in vorteilhafter Weise das Therapieren aller grundsätzlich möglichen, sich bezüglich ihrer Position und/oder Form unterscheidenden Fersenspornen.

Dadurch besteht desweiteren in vorteilhafter Weise bei einer Vielzahl von Behandlungsfällen die Möglichkeit, die Ferse derart hohl zu legen, daß der zu therapierende Fersensporn ohne zusätzliche Nacharbeiten an der Fersensporn-Bettung druckentlastet ist.

Entsprechend einer anderen vorteilhaften Ausführungsform ist das Fersenpolster die im Fersenkissen vorgesehene Ausnehmung formschlüssig eingesetzt und füllt diese vollständig aus. Das Fersenpolster weist Mittel auf, welche ein Herausfallen aus der das Fersenpolster aufnehmenden Ausnehmung verhindern. So ist beispielsweise an der der Ferse zugewandten Seite ein umlaufender, in die Ausnehmung des Fersenkissens eingepaßter Kragen vorgesehen, dessen Dicke sich in radialer Richtung verringert. Dadurch entsteht gleichzeitig zwischen den peripheren Bereichen des Fersenpolsters und der Ausnehmung des Fersenkissens ein Übergang, welcher im wesentlichen kantenfrei ausgebildet ist und eine zusätzliche Druckbe-lastung des Fersengewebes vermeidet, so daß die Kante der Auflage nicht unangenehm spürbar wird.

Um die Fersensporn-Bettung an die individuelle Ausbildung des Fersensporns anpassen zu können, ist Fersenpolster auswechselbar angeordnet. Dazu weist das vorzugsweise aus einem Moosgummi bestehende Fersenkissen einen sich von der Ausnehmung bis zum Rand des Fersenkissens in radialer Richtung erstreckenden Schlitz auf, um die Ausnehmung zum Wechsel des Fersenpolsters besondere Mühe in ausreichendem Maß weiten zu können. Das für das Fersenkissen verwendete Material ist weicher als der für das Fersenkissen eingesetzte Moosgummi und besteht aus Latex oder einem latexartigen Werkstoff, so daß auch durch eine derartige Werkstoffpaarung ein Wechsels des Fersenpolsters erheblich erleichtert wird.

Die Ausnehmung bzw. das Fersenpolster ist von unten nach oben konusartig erweitert, wobei insbesondere ein Böschungs-winkel von im wesentlichen 45° vorgesehen ist, welcher sich insbesondere in Richtung nach vorn bis auf ca. 20° verringert. Die Böschung geht unter weiterer gleichmäßiger Verringerung des Böschungswinkels in die Ebene der Polsteroberfläche über, wodurch hartkantige Übergangsbereiche zwischen Fersenpolster und Fersenkissen in vorteilhafter Weise vermieden werden.

Nach einer zusätzlichen Weiterbildung der Erfindung weist das Fersenpolster einerseits eine geringere Höhe auf als die Ausnehmung im Fersenkissen oder es sind andererseits im Fersenpolster eine zentrale Materialaussparung oder symmetrisch zueinander angeordnete periphere Materialausnehmungen vorgesehen, so daß durch gezielten Austausch des Fersenpolstern eine stufenweise Fersensporn-Therapie durchgeführt werden kann. Für die in dem Fersenpolster vorgesehenen Ausnehmungen ist eine runde oder ovale Form günstig.

Eine schichtförmige Abdeckung des der Ferse zugewandten Seite des Fersenkissen verhindert auf einfache Weise das ein in die Ausnehmung des Fersenkissens eingesetztes Fersenpolster bei Benutzung der Fersensporn-Bettung durch die Bewegung der Ferse beim Laufen herausgedrückt werden kann. Durch einen allseitig geringfügigen Überstand der Abdeckung am Rand des Fersenkissens wird in günstiger Weise eine zusätzliche Druckbelastung des Fersengewebes bei Benutzung der Fersensporn-Bettung vermieden, da das den Rand des Fersenkissens überragende Material der Abdeckung bei Druckbelastung leicht nach unten abgebogen wird und dadurch die Randkante des Fersenkissens verrundet.

Die vorzugsweise durch Kleben mit dem Fersenkissen verbundene Abdeckung besteht aus Leder.

Entsprechend einer bevorzugten Ausführungsform der Erfindung ist die Verwendung von elastischem, gerbstofffreien Zertifikatsleder, vorzugsweise Chevreaux-Leder, vorgesehen, um einerseits die Wirkung des Fersenpolsters so wenig wie möglich einzuschränken und andererseits die schmerzende Ferse nicht zusätzlich durch chemische Substanzen zu belasten.

Um die elastische Nachgiebigkeit des Polsters durch die darübergelegene Lederabdeckung nicht wieder einzuschränken, weist diese nach einer anderen Weiterbildung der Erfindung im Bereich des eingesetzten Fersenpolsters eine Mehrzahl von Schlitzen auf, welche sich im wesentlichen parallel zueinander erstrecken und mit der Symmetrieebene des Fersenkissens einen Winkel von vorzugsweise 45' einschließen. Dadurch wird dieser Bereich der Abdeckung besonders nachgiebig und schränkt die Wirkung des Fersenpolsters nicht ein.

Diese Nachgiebigkeit der Abdeckung im Bereich des eingesetzten Fersenpolsters läßt sich in vorteilhafter Weise erhöhen, indem mehrere, durch Stege getrennte Schlitze in im wesentlichen geradlinig ausgebildeten und sich parallel zueinander erstreckenden Schlitzreihen vorgesehen sind, wobei den Stegen einer Schlitzreihe die Schlitze der sich jeweils neben ihr befindlichen Schlitzreihen gegenüber-liegen.

Für die Behandlung des Fersensporns ist es wichtig, daß die Fersensporn-Bettung die für sie vorgesehene Position in der Fußbekleidung beibehält und beispielsweise bei Belastung nicht verrutscht. Dazu sind auf der der Ferse abgewandten Seite des Fersenkissens eine Mehrzahl von im wesentlichen gleichartig ausgebildeten Noppen vorgesehen. Zur Unterstützung der Wirkung der Noppen ist eine auf der der Ferse abgewandten Seite des Fersenkissens angeordnete Klebfläche günstig. Diese befindet sich an dem den Fußzehen zugewandten Ende der Fersensporn-Bettung und wird nach Entfernen einer abziehbaren Folie nutzbar.

Eine besonders gute Lagestabilität ist durch Verwendung der Fersensporn-Bettung als Bestandteil einer Schuh-Einlegesohle erreichbar.

Entsprechend einer weiteren Ausführungsform der Erfindung ist die Fersensporn-Bettung in den Absatz einer Fußbekleidung eingearbeitet. Die Zugänglichkeit des Fersenkissens bzw. des Fersenpolsters ist durch eine zweitei-lige Ausbildung des Absatzes der Fußbekleidung gesichert. Nach Abschluß der Behandlung des Fersensporns wird das Fersenpolster gegen einen der Größe der Ausnehmung ent-sprechenden Einsatz mit größerer Festigkeit ausgetauscht. Zur Verbindung der beiden schwenkbar miteinander verbundenen Absatzteile sind als Rastelemente ausgebildete Verbindungsmittel vorgesehen.

Vorteilhafte Weiterbildungen der Erfindung sind in den Unteransprüchen gekennzeichnet bzw. werden nachstehend zusammen mit der Beschreibung der bevorzugten Ausführung der Erfindung anhand der Figuren näher dargestellt. Es zeigen:
Figur 1 die bevorzugte Ausführungsform der Erfindung in perspektivischer Darstellung,
Figuren 2a bis 2d verschiedene Ausführungsformen der Erfindung in Ansicht von oben,
Figur 3 die Darstellung der Ansicht eines Schnittes längs der Linie A...A in Figur 5,
Figur 4 die in Figur 1 dargestellte Ausführungsform der Erfindung in Draufsicht,
Figuren 4a und 4b vorteilhafte Varianten der in Figur 4 dargestellten Ausführungsform der Erfindung,
Figur 5 die in Figur 1 dargestellte Ausführungsform der Erfindung in Ansicht von unten,
Figur 6 eine zusätzliche Weiterbildung der in Figur 3 gezeigten Ausführungsform der Erfindung,
Figur 7 eine weitere Ausführungsform der Erfindung, sowie
Figur 8 die Schnittdarstellung eines Details der in Figur 7 gezeigten Ausführungsform der Erfindung.

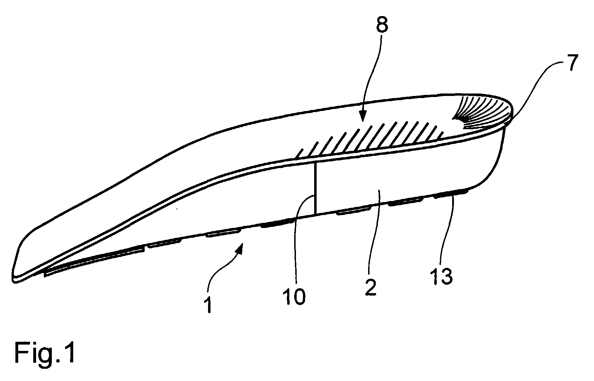

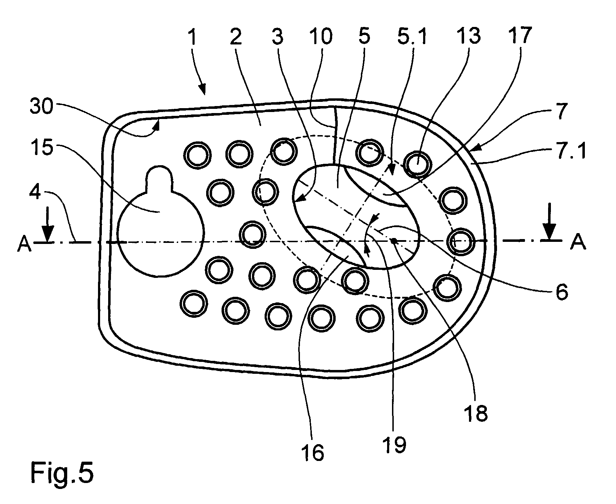

Die in den Figuren 1, 3 und 5 perspektivisch, als Längsschnitt und in Ansicht von unten dargestellte Fersensporn-Bettung 1 weist ein Fersenkissen 2 auf, in welchem eine das Fersenkissen vollständig durchdringende Ausnehmung 3 eingearbeitet ist.

Die an dem Polster 5 vorgesehenen, kragenförmig ausgebildeten Begrenzungsmittel 5.1 verhindern ein Herausfallen des Polsters 5 aus der Ausnehmung 3 nach unten.

Zur mechanischen Entlastung von Teilbereichen der Ferse ist in die Ausnehmung 3 ein Fersenpolster 5 eingesetzt. Das Einsetzen des weicher als das Fersenkissen 2, 2a bis 2d ausgebildeten Fersenpolsters 5 erfolgt formschlüssig, wobei die Ausnehmung 3 bzw. das Fersenpolster 5 eine elliptische Grundfläche mit einer großen Halbachse 6 aufweist.

Die Ausnehmung 3 und das Fersenpolster 5 sind im wesentlichen als flache Scheibe ausgebildet, wobei das Fersenpolster 5 die Ausnehmung 3 im Fersenkissen 2 vollständig ausfüllt.

Der an der der Ferse zugewandten Seite des Fersenpolsters 5 vorgesehene, in die Ausnehmung 3 des Fersenkissens eingepaßten Kragen 5.1 weist eine sich in radialer Richtung von innen nach außen verringernde Dicke auf. Dadurch entsteht zwischen den peripheren Bereichen des Fersenpolsters 5 und der Ausnehmung 3 des Fersenkissens 2 ein Übergang, welcher frei von relativ scharfen Kanten ist und eine zusätzliche Druckbelastung des Fersengewebes vermeidet.

Um das Fersenpolster 5 ohne besondere Mühe auswechseln zu können, ist in dem aus einem Moosgummi bestehende Fersenkissen 2 ein sich von der Ausnehmung 3 bis zum Rand des Fersenkissens 2 in radialer Richtung erstreckenden Schlitz 10 vorgesehen. Durch diesen Schlitz kann die Ausnehmung 3 zum Wechseln des Fersenpolsters 5 in ausreichendem Maße geweitet werden. Das für das Fersenpolster 5 verwendete Material ist weicher als der für das Fersenkissen 2 eingesetzte Moosgummi. Das Fersenpolster 5 besteht aus Latex oder einem latexartigen Werkstoff, so daß auch durch diese Werkstoffpaarung ein Wechseln des Fersenpolsters 5 erleichtert wird.

Durch Variation der Größe und Form der an Unterseite des Polsters 5 symmetrisch angeordneten Materialaussparungen 16, 17 ist eine Anpassung des Druckentlastungsbereichs der Fersensporn-Bettung an die individuelle Ausbildung des Fersensporns möglich.

Die schichtförmige Abdeckung 7 des der Ferse zugewandten Seite des Fersenkissen 2 besteht aus einem elastischen, gerbstofffreien Chevreaux-Leder und durch Kleben mit dem Fersenkissen 2 verbunden. Die Abdeckung 7 hält das Fersenpolster 5 in der ihm zugedachten Position. Der allseitig vorgesehene, geringfügige Überstand 7.1 der Abdeckung 7 am Rand des Fersenkissens 2 wird bei Druckbelastung leicht nach unten abgebogen und verrundet dadurch die Randkante des Fersenkissens 2.

Die auf der der Ferse abgewandten Seite des Fersenkissens 2 vorgesehenen gleichartig ausgebildeten Noppen 13 halten die Fersensporn-Bettung 1 in der ihr zugedachten Position innerhalb der Fußbekleidung und verhindern ein Verrutschen bei der beim Gehen oder Stehen auftretenden mechanischen Belastung. Zur Unterstützung der Wirkung der Noppen ist eine auf der der Ferse abgewandten Seite des Fersenkissens 2 angeordnete Klebfläche 14 vorgesehen. Sie befindet sich an dem den Fußzehen zugewandten Ende der Fersensporn-Bettung 1 und ist mit einer abziehbaren Folie 15 bedeckt.

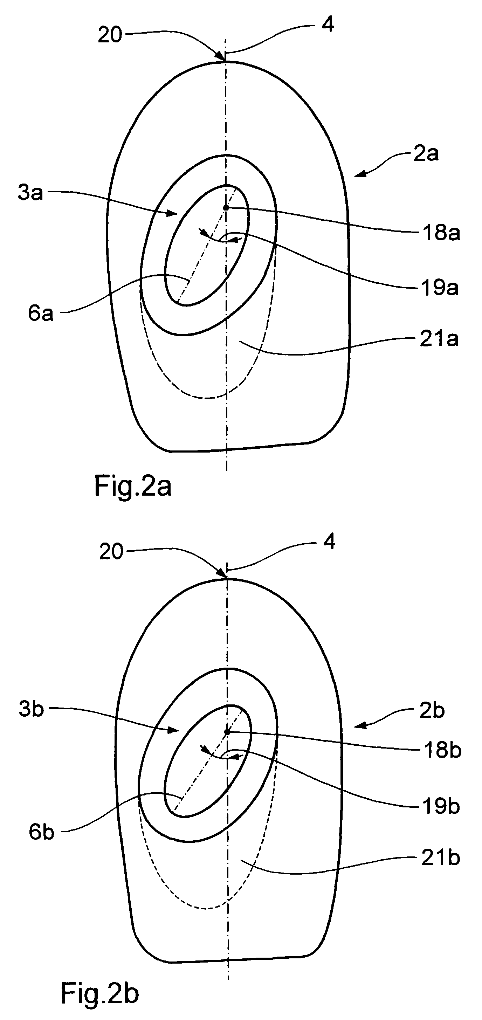

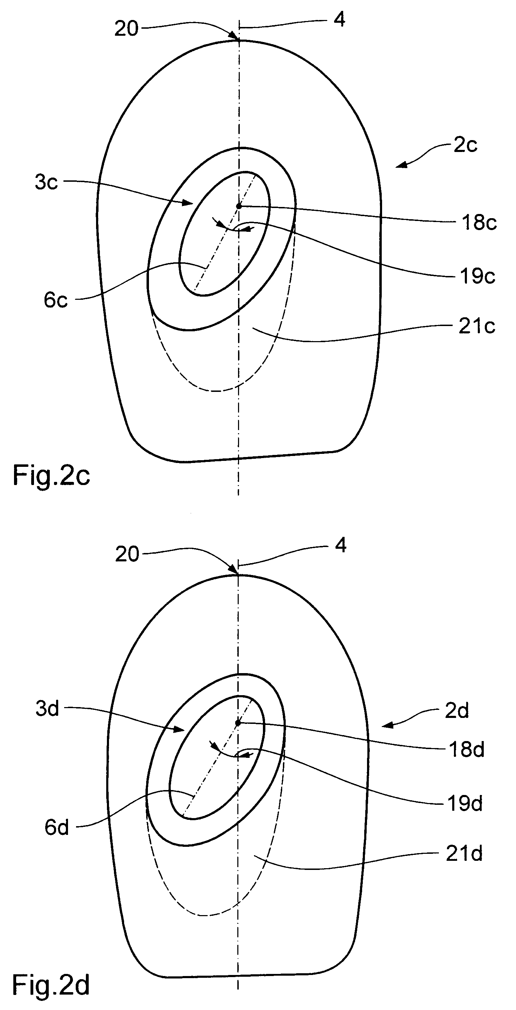

Die Anordnungsgeometrie der elliptischen Ausnehmung 3 bzw. des elliptischen Polsters 5 wird durch die Position des Schnittpunktes 18 und die Größe des Winkels 19 zwischen der Fußmittelachse 4 und der großen Achse 6 der Ellipse bestimmt und in den Figuren 2a bis 2d erläutert.

Die Figuren 2a bis 2d zeigen verschiedene Ausführungsformen 2a, 2b, 2c, 2d des Fersenkissens von Fersenspornbettung für unterschiedliche Schuhformen.

Die Form der elliptischen Ausnehmungen 3a, 3b, 3c, 3d bzw. des in diese Ausnehmungen einsetzbaren Fersenpolsters ist so gewählt, daß das Verhältnis der Länge der großen Achse 6a, 6b, 6c, 6d zur kleinen Achse der Ellipse einen Wert von etwa 1,7 aufweist.

Gleichermaßen ist bei den ellipsenartigen Ausnehmungen 3a, 3b, 3c, 3d bzw. Polstern 5 der vorn gelegene Brennpunkt der Ellipse zur Fußinnenseite hin versetzt angeordnet, wobei die große Achse 6a, 6b, 6c, 6d der Ellipse die Fußmittelachse 4 im Bereich ihres hinteren Viertels bei 20% ihrer Länge schneidet und der Schnittpunkt 18a, 18b, 18c, 18d zwischen Fußmittelachse 4 und großer Achse 6a, 6b, 6c, 6d der Ellipse vom hinteren Ende 20 des Fußes im wesentlichen um die Länge der großen Achse der Ellipse nach vorn versetzt angeordnet ist.

Bei den gezeigten Ausführungsformen schließen die große Achse 6a, 6b, 6c, 6d der Ellipse und die Mittelachse 4 des Fußes - bedingt durch die vorstehend beschriebene Lage des vorn gelegenen Ellipsenbrennpunktes - einen Winkel 19a, 19b, 19c, 19d von etwa 45°ein.

Die Ausnehmungen 3a, 3b, 3c, 3d erweitern sich von unten nach oben konusartig mit einem Böschungswinkel von etwa 45°, wobei sich die Neigung der Böschung 21a, 21b, 21c, 21d in Richtung nach vorn bis auf etwa 20° verringert und unter weiterer gleichmäßiger Verringerung in die von der Polsteroberfläche aufgespannten Ebene übergeht.

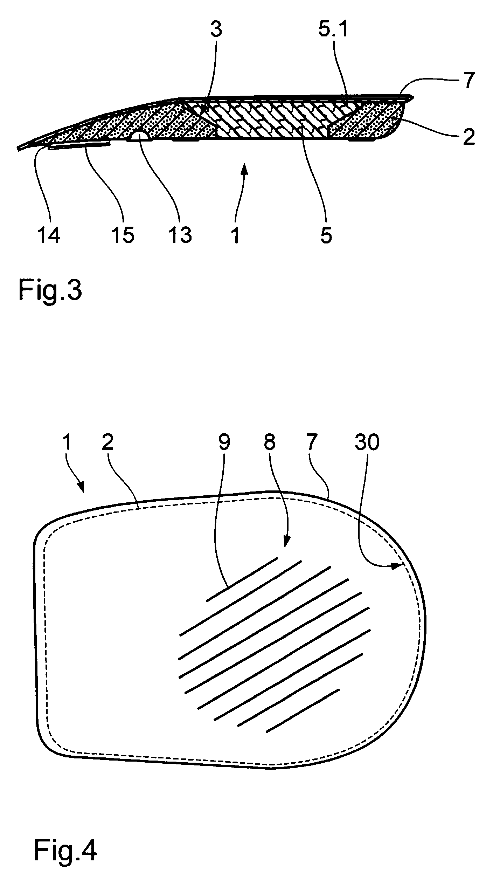

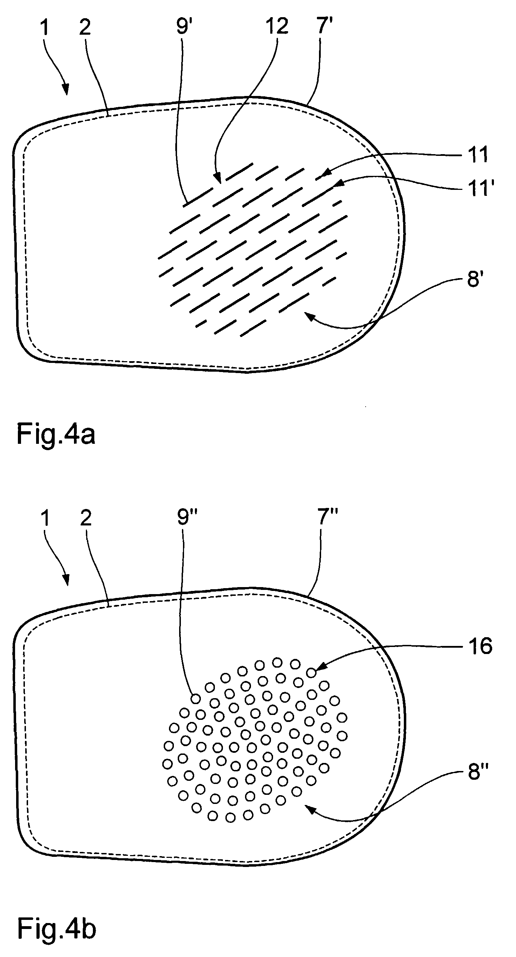

In den Figuren 4, 4a und 4b sind unterschiedliche Ausführungsformen von Fersensporn-Bettungen 1 in Draufsicht dargestellt.

Die Lederabdeckung 7 des Fersenkissens 2 weist entsprechend Figur 4 im Bereich 8 des eingesetzten Fersenpolsters 5 eine Mehrzahl von Schlitzen 9 auf, welche sich im wesentlichen parallel zueinander erstrecken. Die Schlitze 9 schließen mit der Längsachse des Fersenkissens 2 einen Winkel von im wesentlichen 45° ein. Durch die Schlitze 9 wird der Bereich 8 der Abdeckung 7 besonders nachgiebig und schränkt dadurch die schmerzlindernde Wirkung des Fersenpolsters 5 nicht ein.

Bei der in Figur 4a dargestellten Fersensporn-Bettung 1 weist die an der Oberseite des Fersenkissens 2 durch Kleben befestigte Abdeckung 7 im Bereich 8' des eingesetzten Fersenpolsters eine Mehrzahl von Schlitzen 9' auf, welche durch Stege 12 getrennt, in im wesentlichen geradlinig ausgebildeten und sich parallel zueinander erstreckenden Schlitzreihen 11, 11' angeordnet sind. Da den Stegen 12 einer Schlitzreihe 11 die Schlitze 9' der sich jeweils neben ihr befindlichen Schlitzreihen 11' gegenüberliegen, sichert diese Schlitzanordnung eine besonders hohe Elastizität der Abdeckung 7' im Bereich 8'.

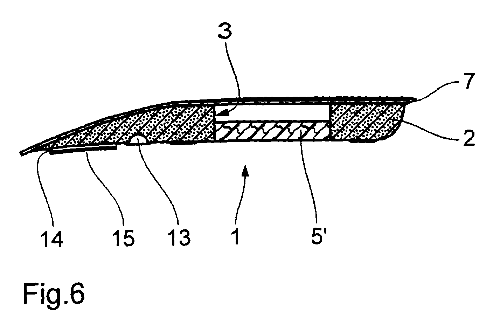

Die Schnittdarstellung gemäß Figur 6 zeigt eine Fersensporn-Bettung 1 mit einem Fersenpolster 5', welches eine geringere Höhe aufweist als die Ausnehmung 3 im Fersenkissen 2. Die Veränderung der Höhendifferenz zwischen Ausnehmung 3 und Fersenpolster 5' gestattet eine Anpassung des Druckentlastungsbereichs der Fersensporn-Bettung 1 an die individuelle Ausbildung des Fersensporns.

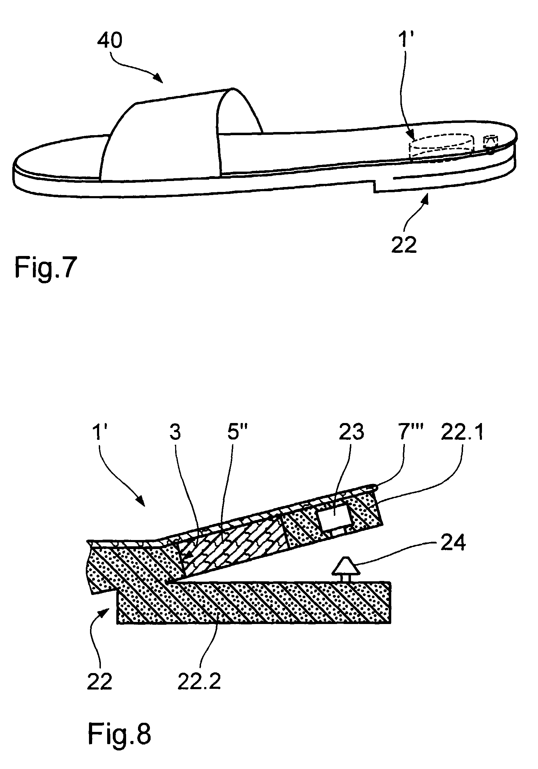

Die in Figur 7 in Seitenansicht gezeigte sandalenförmige Fußbekleidung 20 weist eine in den Sandalettenabsatz 21 eingesetzte Fersensporn-Bettung 1' auf.

In Figur 8 ist die Ansicht eines Längsschnittes durch den zweiteilig ausgebildeten Absatz 21 der Fußbekleidung 20 dargestellt. Das Absatzteil 21.1 bildet die Lauffläche des Absatzes und ist einseitig schwenkbar an das Fersenkissen der Fersensporn-Bettung 1' bildende Absatzteil 21.2 angelenkt. Das in die Ausnehmung 3 eingesetzte Fersenpolster 5'' ist durch Abschwenken der Lauffläche (Figur 9) zugänglich und kann bei Bedarf, beispielsweise nach Abschluß der Behandlung des Fersensporns, durch einen der Härte des Fersenkissens 21.1 entsprechenden Einsatz ausgetauscht werden. Die an dem abgeschwenkten Ende der Lauffläche 21.2 vorgesehenen Mittel zu dessen Befestigen an dem Fersenkissen 21.1 sind als Rastelemente ausgebildet.

Die Erfindung beschränkt sich in ihrer Ausführung nicht auf das vorstehend angegebene bevorzugte Ausführungsbeispiel. Vielmehr ist eine Anzahl von Varianten denkbar, welche von der dargestellten Lösung auch bei grundsätzlich anders gearteten Ausführungen Gebrauch macht. Die in der vorangehenden Beschreibung genannten geometrischen Angaben dienten lediglich der Verdeutlichung des Ausführungsbeispiels, schränken aber die Erfindung nicht ein. Konstruktive Richtlinie ist die Zusammenfassung von unterschiedlich gelagerten Fersensporn-Druckstellen zu einem zusammengefaßten Bereich, dessen geometrische Form sich aus der aufgrund der ärztlichen Erfahrung resultierenden geometrischen Überlagerung ergibt.

## Patentansprüche

1. Fersensporn-Bettung (1, 1') als Teil eines Fersenkissens (2, 2a, 2b, 2c, 2d, 22.1) welches seinerseits gegebenenfalls Teil des Fußbetts einer orthopädischen Einlage oder eines Schuhs ist, bestehend aus einem elastischen Werkstoff, welches zur mechanischen Entlastung eines Teilbereichs der Ferse eine Ausnehmung (3, 3a, 3b, 3c, 3d) aufweist, in welche ein Polster (5, 5', 5'') zur Aufnahme des Fersensporns eingefügt ist, wobei der Werkstoff des Fersenkissens bei Druckbelastung nachgiebiger ist als der an das Polster angrenzende Werkstoff,
**dadurch gekennzeichnet**,
daß die Außenabmessungen des Polsters (5, 5', 5'') bzw. der Ausnehmung (3, 3a, 3b, 3c, 3d) in Längsrichtung des Fußes größer sind als in Querrichtung, wobei der vordere Teil der Ausnehmung zur Fußinnenseite hin weist, in der Weise, daß mindestens der überwiegende Teil der Fersenspornausbildungen mit einer einzigen und geschlossenen geometrischen Form der Ausnehmung druckentlastend erfaßbar ist.

2. Fersensporn-Bettung nach Anspruch 1, **dadurch gekennzeichnet**, daß das Polster (5, 5', 5'') bzw. die Ausnehmung (3, 3a, 3b, 3c, 3d) eine symmetrisch verrundete, insbesondere elliptische oder ovale, Grundfläche aufweist, wobei das Verhältnis der Länge der großen Achse (6, 6a, 6b, 6c, 6d) zur kleinen Achse der Ellipse einen Wert zwischen 1,4 bis 2,0, vorzugsweise 1,7, aufweist.

3. Fersensporn-Bettung nach Anspruch 2, **dadurch gekennzeichnet**, daß bei einer ellipsenartigen oder ovalen Ausgestaltung der Ausnehmung (3, 3a, 3b, 3c, 3d) bzw. des Polsters (5, 5', 5'') der vorn gelegene Brennpunkt der Ellipse bzw. Mittelpunkt des Halbkreises des Ovals zur Fußinnenseite hin versetzt angeordnet ist.

4. Fersensporn-Bettung nach Anspruch 3, **dadurch gekennzeichnet**, daß die große Achse (6, 6a, 6b, 6c, 6d) der Ellipse bzw. Längsachse des Ovals die Fußmittelachse (4) im Bereich ihres hinteren Viertels, insbesondere bei im wesentlichen 20% ihrer Länge, schneidet.

5. Fersensporn-Bettung nach Anspruch 4, **dadurch gekennzeichnet**, daß der Schnittpunkt (18, 18a, 18b, 18c, 18d) zwischen Fußmittelachse und großer Achse (6, 6a, 6b, 6c, 6d) der Ellipse bzw. Längsachse des Ovals vom hinteren Ende (20) des Fußes im wesentlichen um die Länge der großen Achse der Ellipse bzw. das Maß der Längserstreckung des Ovals nach vorn versetzt angeordnet ist.

6. Fersensporn-Bettung nach Anspruch 2, **dadurch gekennzeichnet**, daß die große Achse (6, 6a, 6b, 6c, 6d) der Ellipse bzw. die Längsachse des Ovals und die Mittelachse (4) des Fußes einen Winkel (19, 19a, 19b, 19c, 19d) von 35° bis 55°, vorzugsweise von 45°, einschließen.

7. Fersensporn-Bettung nach einem der vorangehenden Ansprüche, **dadurch gekennzeichnet**, daß an dem Polster (5, 5', 5'') Begrenzungsmittel (5.1) vorgesehen sind, welche ein Herausfallen des Polsters (5, 5', 5'') aus der Ausnehmung (3, 3a, 3b, 3c, 3d) verhindern.

8. Fersensporn-Bettung nach einem der vorangehenden Ansprüche, **dadurch gekennzeichnet**, daß die Ausnehmung (3, 3a, 3b, 3c, 3d) bzw. das Polster (5, 5', 5'') von unten nach oben konusartig, insbesondere mit einem Böschungswinkel von im wesentlichen 45° erweitert, wobei insbesondere in Richtung nach vorn die Neigung der Böschung (21a, 21b, 21c, 21d) bis auf ca. 20° verringert ist und unter weiterer gleichmäßiger Verringerung in die Ebene der Polsteroberfläche übergeht.

9. Fersensporn-Bettung nach einem der vorangehenden Ansprüche, **dadurch gekennzeichnet**, daß das Polster (5, 5', 5'') auswechselbar ausgebildet ist, wobei insbesondere Polster unterschiedlicher Elastizität und/oder mit einer unterschiedlich großen zentralen oder peripher symmetrisch an der Unterseite angeordneten Materialaussparungen (16, 17) gegeneinander austauschbar sind.

10. Fersensporn-Bettung nach einem der vorangehenden Ansprüche, **dadurch gekennzeichnet**, daß das Fersenkissen (2, 2a, 2b, 2c, 2d) aus Moosgummi, insbesondere mit einer Härte (Shore A) von im wesentlichen 30 besteht.

11. Fersensporn-Bettung nach einem der vorangehenden Ansprüche, **dadurch gekennzeichnet**, daß das Fersenkissen (2, 2a, 2b, 2c, 2d) einen sich von der Ausnehmung (3, 3a, 3b, 3c, 3d) bis zum Rand des Fersen-kissens in radialer Richtung erstreckenden Schlitz (10) zum erleichterten Einsetzen und Herausnehmen des Polsters aufweist.

12. Fersensporn-Bettung nach einem der vorangehenden Ansprüche, **dadurch gekennzeichnet**, daß das Fersenkissen (2, 2a, 2b, 2c, 2d) aus Latex oder einem entsprechenden Werkstoff, insbesondere mit einer Härte (Shore A) von im wesentlichen weniger als 10, besteht.

13. Fersensporn-Bettung nach einem der vorangehenden Ansprüche, **dadurch gekennzeichnet**, daß das Fersenkissen (2, 2a, 2b, 2c, 2d) eine Abdeckung (7, 7', 7'', 7''') als Fußauflage aufweist.

14. Fersensporn-Bettung nach Anspruch 13, **dadurch gekennzeichnet**, daß die Abdeckung aus einem gerbstofffreiem Zertifikatsleder besteht.

15. Fersensporn-Bettung nach einem der Ansprüche 13 oder 14, **dadurch gekennzeichnet**, daß die Abdeckung (7, 7', 7'') in dem das Polster (5, 5') bedeckenden Bereich (8, 8'), insbesondere runde, Durchbrüche oder Schlitze (9, 9', 9'') aufweist.

16. Fersensporn-Bettung nach Anspruch 15, **dadurch gekennzeichnet**, daß mehrere, durch Stege (11) getrennte Schlitze (9') in im wesentlichen geradlinig ausgebildeten und sich parallel zueinander erstreckenden Schlitzreihen (12) angeordnet sind, wobei den Stegen (11) einer Schlitzreihe (12) die Schlitze (9') der sich jeweils neben ihr befindlichen Schlitzreihen gegenüberliegen.

17. Fersensporn-Bettung nach einem der vorangehenden Ansprüche, **dadurch gekennzeichnet**, daß auf der der Ferse abgewandten Oberfläche des Fersenkissens (2, 2a, 2b, 2c, 2d) Noppen (13) vorgesehen sind.

18. Fersensporn-Bettung nach einem der vorangehenden Ansprüche als Teil einer Fußbekleidung, **dadurch gekennzeichnet**, daß der Absatz (22) zweiteilig ausgebildet ist, wobei das die Lauffläche des Absatzes (22) bildende Teil (22.2) an das das Fersenkissen bildende Teil (22.1) schwenkbar angelenkt ist und zur lösbaren Verbindung der Absatzteile (22.1, 22.2) als Rastelemente ausgebildete Verriegelungsmittel (23, 24) vorgesehen sind.
